# NEW EUROPEAN PATENT SPECIFICATION

(11) **EP 0 474 096 B2**
(45) Date of publication and mention of the opposition decision: **16.05.2001**
(45) Mention of the grant of the patent: 29.11.1995
(21) Application number: 91114352.7
(22) Date of filing: 27.08.1991
(51) Int. Cl.: C07C 227/40, C07C 229/36, C07C 229/02, C07C 229/56

(54) **Method for extracting amino acid ester**
Verfahren zur Extraktion eines Aminosäureesters
Procédé pour l'extraction d'un ester d'un amino-acide

(30) Priority: 03.09.1990 JP 23064390
(43) Date of publication of application: 11.03.1992
(73) Proprietor: MITSUI CHEMICALS, INC., Tokyo (JP)
(72) Inventor: Kamashita, Tomoko, Ohmuta-shi, Fukuoka-ken (JP); Yamashita, Hiroyuki, Ohmuta-shi, Fukuoka-ken (JP); Nagata, Teruyuki, Ohmuta-shi, Fukuoka-ken (JP); Ajioka, Masanobu, Yokohama-shi, Kanagawa-ken (JP)
(74) Representative: Luderschmidt, Schüler & Partner GbR

(56) References cited:
- DE-A- 1 643 267
- DE-A- 2 528 069
- US-A- 3 786 039
- US-A- 4 680 403
- D.D. PERRIN, "Dissociation Constants of Organic Bases in Aqueous Solution", International Union of Pure and Applied Chemistry, Analytical Chemistry Division Commission on Electroanalytical Chemistry, published as a supplement to Pure and Applied Chemistry, Butterworths, London, 1965, p. 395

## Description

The present invention relates to a method for extracting a free amino acid ester efficiently from a hydrous solution containing a mineral acid salt of said amino acid ester, using an organic solvent.

Amino acid esters are important as an intermediate used in peptide synthesis. Methyl L-phenylalaninate, in particular, have recently drawn attention as a main raw material for Aspartame, an artificial sweetening.

Methods for esterification of an amino acid have been known from old times, and there is ordinarily used a method of introducing hydrogen chloride gas into an alcohol suspension of an amino acid or a method of adding concentrated sulfuric acid to said suspension.

The thus produced amino acid ester is isolated in the form of a salt such as hydrochloride and sulfate. In many cases, the salt is made into an aqueous solution, a base is added to the aqueous solution to effect neutralization to liberate an amino acid ester, the amino acid ester is extracted with an organic solvent, and the resulting organic solvent solution per se is used in peptide synthesis. The reason for this is that amino acid esters tend to cause self-condensation to form a polypeptide or a diketopiperazine derivative and accordingly have a problem in stability while their mineral acid salts have excellent storage stability.

When a free amino acid ester is needed, the above practice of isolating it in the form of a mineral acid salt is not advantageous from the viewpoints of the yield and operation. ---- US-A-4 680 403 which corresponds to Japanese Patent Application Laid-Open No. 267600/1986 discloses a process which comprises esterifying L-phenylalanine in methanol in the presence of a strong acid, then adding thereto an aqueous basic solution (for neutralization of the catalyst) and an organic solvent (for extraction of free methyl L-phenylalaninate) immiscible with water, separating the organic solvent solution, and condensing the methyl L-phenylalaninate dissolved in the separated organic solvent layer, with a N-protected L-aspartic acid anhydride. This process is advantageous in that it contains no step for separation of amino acid hydrochloride or sulfate. However, the document states that the process gives no good yield of the condensation.

DE-A-2528069 discloses a method for extracting an amino acid ester, which comprises adding a base (sodium carbonate) to an aqueous solution of a mineral acid salt of an amino acid ester (p-nitrobenzyl-2-methyl-1-phenylalanine ester hydrobromide) to bring the pH of the solution to 8, then adding a water insoluble organic solvent (ethyl acetate) and transferring the liberated amino acid ester into the organic layer.

In the above both cases of (1) isolating a mineral acid salt of an amino acid ester, neutralizing the salt in its aqueous solution and extracting an amino acid ester and (2) esterifying an amino acid in the presence of an acid and subjecting the reaction mixture to neutralization and extraction to obtain an organic solvent solution containing an amino acid ester, there is a problem of hydrolysis of amino acid ester during neutralization and extraction. However, no sufficient solution to this problem has been made hitherto.

### Summary of the Invention

The object of this invention is to provide an improved method for extracting an amino acid ester, which process comprises adding a water-insoluble organic solvent to a hydrous solution containing a mineral acid salt of an amino acid ester, then adding a base thereto to effect neutralization to liberate the amino acid ester, and transferring the amino acid ester into the organic layer.

The above object has been achieved by keeping the pH of the aqueous layer at 7-8 and the temperature at 0-50°C. during the extraction.

The above object has also been achieved by carrying out the extraction a plurality of times and keeping the pH of the aqueous layer at 7-8 and the temperature at 0-50°C. at each extraction.

The present invention makes it possible to, in solvent extraction of an amino acid ester from an hydrous solution containing a mineral acid salt of said amino acid ester, minimize the reduction in yield caused by hydrolysis of said amino acid ester and obtain said amino acid ester efficiently in the form of an organic solvent solution.

### Detailed Description of the Invention

In order to minimize the hydrolysis of an amino acid ester in the step for preparing an organic solvent solution containing the amino acid ester from a hydrous solution containing a mineral acid salt of the amino acid ester, it is necessary to (1) add a water-insoluble organic solvent to the hydrous solution containing a mineral acid salt of the amino acid ester, then (2) add a base thereto to effect neutralization and liberate the amino acid ester, and (3) quickly transfer the amino acid ester into the organic layer to minimize the time of contact of the amino acid ester with the aqueous layer.

The present inventors made study on the rate of hydrolysis of amino acid ester in water. As a result, it was found that the pH of aqueous layer is the most important factor for the hydrolysis rate and that (1) when the pH is higher than 8, the hydrolysis of amino acid ester occurs quickly, but, when the pH is 8 or lower, sufficient stability for the hydrolysis is secured in the industrial production of amino acid ester and (2) the extraction efficiency by water-insoluble organic solvent is lower in terms of distribution ratio when the ρH is lower and no practical extraction is obtained when the pH is lower than 7.

The above finding has led to the completion of a method which can minimize the hydrolysis of amino acid ester and which enables the efficient extraction of amino acid ester.

The present invention resides in a method for extracting an amino acid ester, which method comprises adding a water-insoluble organic solvent to a hydrous solution containing a mineral acid salt of an amino acid ester, then adding a base thereto to liberate the amino acid ester, and transferring the amino acid ester into the organic layer, wherein the extraction is carried out a plurality of times, the pH of the aqueous layer being kept at 7-8 and the temperature at 0-50 °C in each extraction.

When the extraction of an amino acid ester from its aqueous layer is carried out a plurality of times in order to obtain a higher extraction efficiency, if the pH adjustment to 7-8 is made only at the first extraction and no pH adjustment by the addition of a base is made at the second and later extractions, the pH of the aqueous layer gets gradually lower and the extraction efficiency decreases as the times of extraction increase. The reason is presumed as follows: that is, at pH 7-8, the mineral acid salt of an amino acid ester is not completely decomposed into the amino acid ester and there exist, in the aqueous layer, said mineral acid salt (which is acidic) and an amino acid ester (which is basic) liberated from said salt; at the subsequent extraction(s), the amino acid ester is transferred into the organic layer and the mineral acid salt remains in the aqueous layer, whereby the aqueous layer gets acidic and the pH gets lower.

The amino acid ester used in the present invention may be a racemic modification or an optical active substance. Its examples include alkyl or aromatic (e.g. methyl, ethyl, phenyl, benzyl) esters of neutral α-amino acids (e.g. glycine, alanine, valine, leucine, isoleucine, phenylalanine, serine, threonine), basic α-amino acids (e.g. lysine, alginine), acidic α-amino acids (e.g. aspartic acid, glutamic acid), *β*-phenylalanine, β-aminopropionic acid, γ-aminobutyric acid and aminobenzoic acid.

The mineral acid in the mineral acid salt of an amino acid ester used in the present invention is hydrochloric acid, sulfuric acid, nitric acid or phosphoric acid.

In the present invention, the hydrous solution containing a mineral acid salt of an amino acid ester includes not only an aqueous solution of said mineral acid salt but also a solution obtained by esterifying an amino acid in an alcohol in the presence of a mineral acid and adding water to the reaction mixture. In the hydrous solution, it is not necessary that the mineral acid salt of an amino acid ester be completely dissolved therein. The water in the solution obtained by adding water to the reaction mixture, may be a water after the addition of a water-insoluble organic solvent or a water added in the form of an aqueous base solution. The amount of water added is preferably about 5-50 times the weight of the mineral acid to be neutralized. When the amount of water is small, the inorganic salt generated by an acid-base reaction is precipitated, making difficult the separation operation. When the amount of water is large, the proportion of the amino acid ester distributed into the aqueous layer is large, reducing the extraction efficiency and the volume efficiency.

The water-insoluble organic solvent used as an extraction solvent in the present invention, can be any as long as it can be separated from the aqueous layer containing the inorganic salt generated by an acid-base reaction. As such a solvent, there can be mentioned, for example, hydrocarbons such as benzene, toluene, hexane and cyclohexane; halogenated hydrocarbons such as dichloromethane, chloroform, carbon tetrachloride, 1,2-dichloroethane and chlorobenzene; ethers such as ethyl ether, butyl ether, tetrahydrofuran and dioxane; and esters such as ethyl acetate, butyl acetate and methyl propionate. A halogenated hydrocarbon or an ester is preferable because of the higher extraction efficiency.

The amount of water-insoluble organic solvent used in one extraction operation is preferably about 0.1-10 times the weight of the hydrous solution containing a mineral acid salt of an amino acid ester. While the percent extraction is higher as the amount of water-insoluble organic solvent used is larger, the use of the solvent in an amount larger than 10 times is disadvantageous industrially in view of the concentration of amino acid ester in extract, the volume efficiency and the solvent recovery. The use of the solvent in an amount smaller than 0.1 time makes large the amount of amino acid ester remaining in the aqueous layer, making it necessary to increase the times of extraction.

The base used in the present invention includes inorganic bases typified by alkali metal hydroxides such as sodium hydroxide and potassium hydroxide, alkali metal hydrogencarbonates such as sodium hydrogencarbonate and potassium hydrogencarbonate, and alkali metal carbonates such as sodium carbonate and potassium carbonate, as well as organic bases typified by tertiary amines such as triethylamine and tributylamine. An inorganic base is preferable because it is not taken into the organic solvent layer containing an amino acid ester.

Preferably, the base is added as it is or in the form of an aqueous solution while the mixture of a hydrous solution containing a mineral acid salt of an amino acid ester with a water-insoluble organic solvent is being stirred. When a strong base, for example, an alkali metal hydroxide is used, it is preferably added dropwise in the form of a 20% or lower water solution in order to suppress the hydrolysis of amino acid ester caused by local pH increase.

With respect to the temperature employed during extraction, a lower temperature gives slower hydrolysis of amino acid ester. However, extraction can be preferably carried out at 0-50 C as long as the pH of the aqueous layer is kept at 7-8. A temperature lower than 0°C is not practical because the aqueous solution may be solidified. A temperature higher than 50°C is not preferable because the amino acid ester is hydrolyzed easily.

The pH of the aqueous layer during extraction is 7-8 as mentioned above. A pH higher than 8 gives a higher percent extraction but incurs sharp increase in hydrolysis of a amino acid ester. A pH lower than 7 causes no hydrolysis of amino acid ester, but reduces percent extraction significantly and is not practical.

The present invention is hereinafter described in detail by way of Examples. However, the present invention is not restricted to these Examples.

### Reference Examples 1-11 and Comparative Examples 1-18

0.1 mole of a mineral acid salt of an amino acid ester, shown in Table 1 was dissolved or suspended in 30 g of water. Thereto was added 60 g of 1,2-dichloroethane. While the mixture was kept at 20-25°C and stirred, a 20% water solution of sodium hydroxide was dropwise added thereto in 0.5 hour, and then stirring and extraction were carried out for 0.5 hour while the pH of the aqueous layer was kept at a constant level in the range of 6.5-8.5. The organic layer was separated from the aqueous layer. Then, there were measured percent extraction of fed amino acid ester in organic layer, percent remaining of said ester in aqueous layer, and percent hydrolysis of said ester in aqueous layer (the hydrolyzed ester remains in the aqueous layer as an amino acid). The results are shown in Table 1.

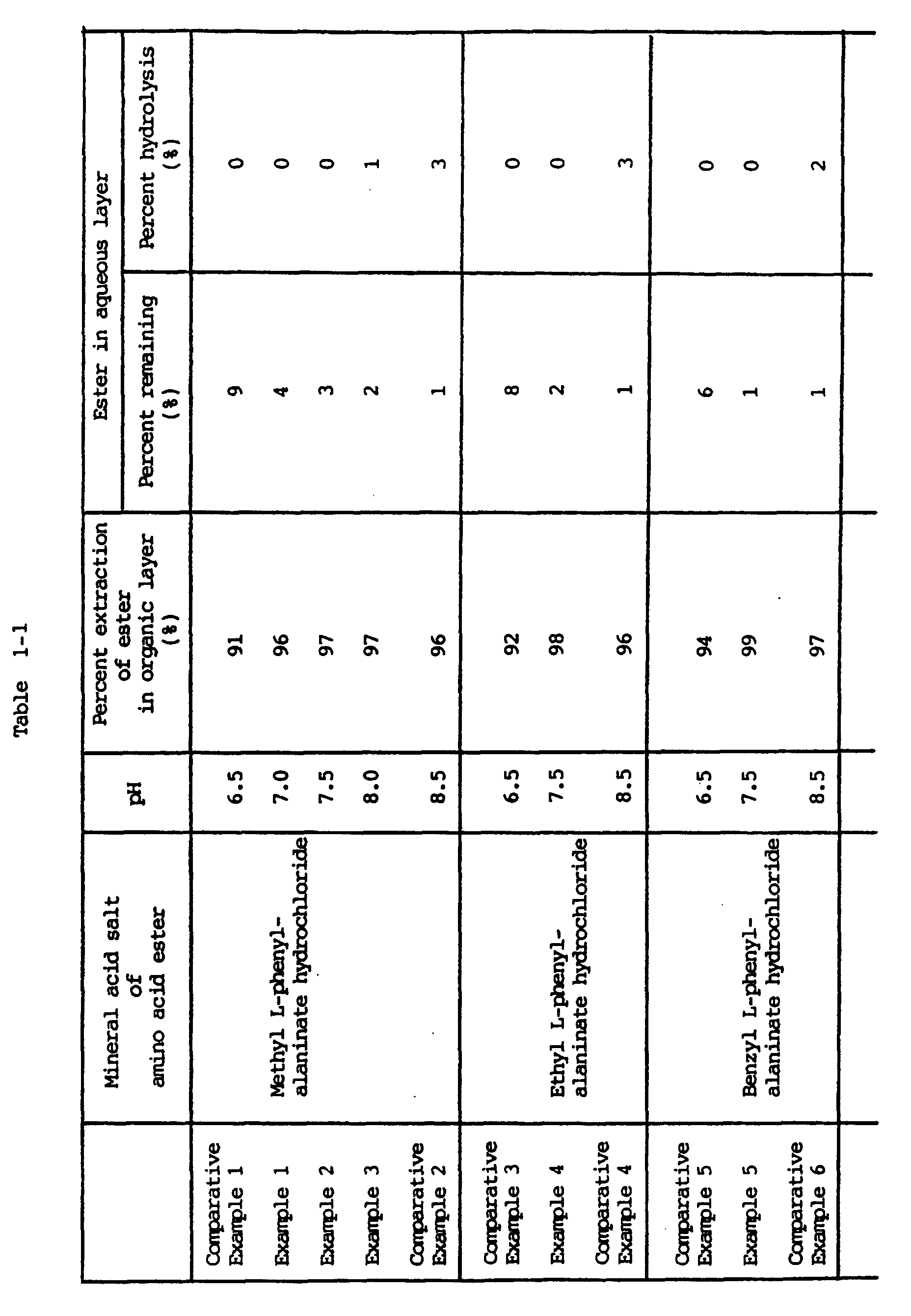

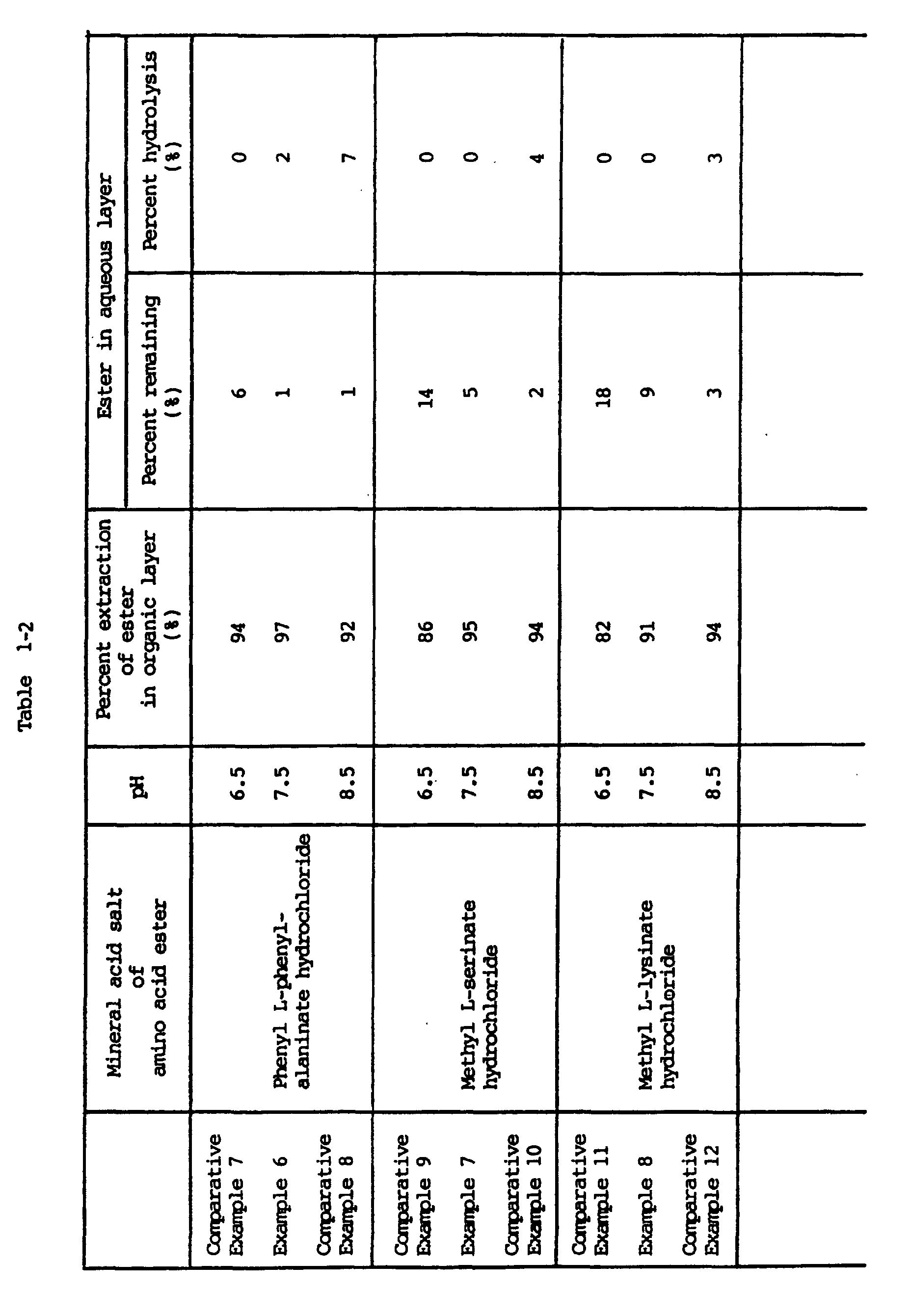

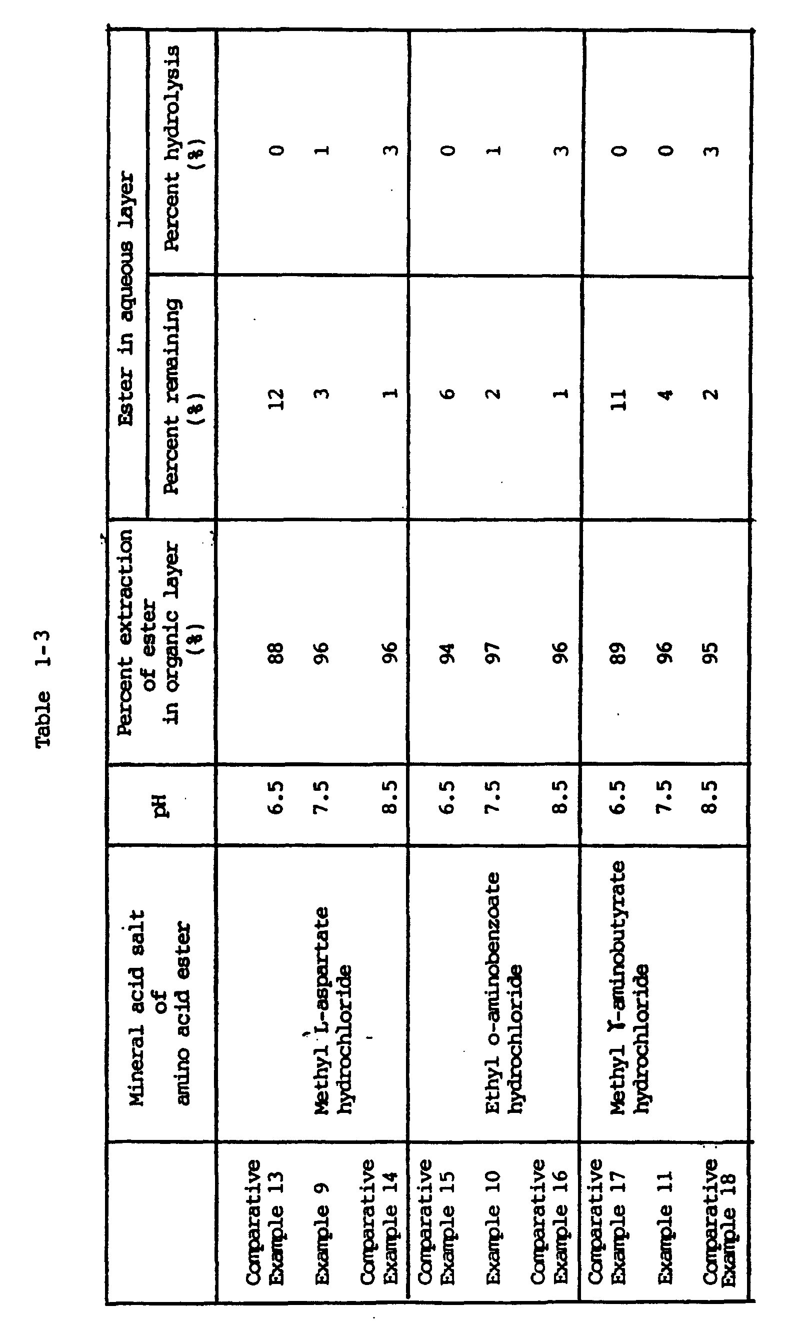

### Example 12

36 g of 98% sulfuric acid was added to a suspension of 33.1 g of L-phenylalanine in 60 g of methanol, and a reaction was carried out at 40-50°C for 7 hours. The reaction mixture contained 34.6 g of methyl L-phenylalaninate and 1.2 g of unreacted L-phenylalanine. Thereinto was fed 70 g of 1,2-dichloroethane, and about 220 g of a 8% water solution of sodium hydroxide was dropwise added in 1.5 hours with stirring at 20-25°C. Successively, stirring and extraction were carried out for 0.5 hour while the pH of the aqueous layer was kept at 7.5. The organic layer was separated from the aqueous layer. The organic layer contained 29.4 g (percent extraction = 85%) of methyl L-phenylalaninate, and the aqueous layer contained 4.8 g of methyl L-phenylalaninate and 1.6 g of L-phenylalanine. Therefore, the percent hydrolysis of methyl L-phenylalaninate during extraction was only 1.2%.

70 g of 1,2-dichloroethane was added to the aqueous layer. Thereto was dropwise added a 8% water solution of sodium hydroxide in 10 minutes with stirring. Successively, stirring and second extraction were carried out for 0.5 hour while the pH of the aqueous layer was kept at 7.5. The organic layer was separated from the aqueous layer. The organic layer contained 4.0 g (percent extraction = 83%) of methyl L-phenylalaninate, and the aqueous layer contained 0.75 g of methyl L-phenylalaninate and 1.65 g of L-phenylalanine.

The aqueous layer after the second extraction was subjected to the same procedure as in the second extraction, to carry out a third extraction. The organic layer contained 0.62 g (percent extraction = 83%) of methyl L-phenylalaninate, and the aqueous layer contained 0.12 g of methyl L-phenylalaninate and 1.66 g of L-phenylalanine.

After the third extraction, the total organic layer contained 34.02 g (percent extraction = 98%) of methyl L-phenylalaninate and the percent hydrolysis of methyl L-phenylalaninate was only 1.3%.

The results are shown in Table 2.

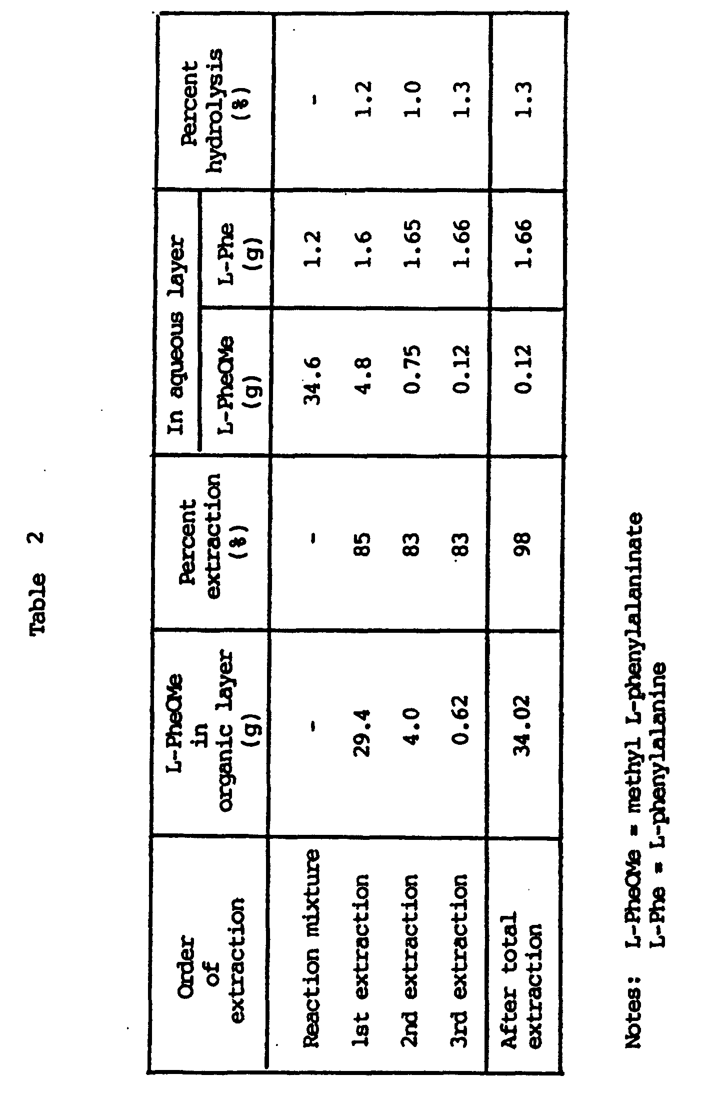

### Example 13

The same extraction procedure as in Example 12 was repeated except that the 1,2-dichloroethane used in Example 12 was changed to toluene. The results are shown in Table 3.

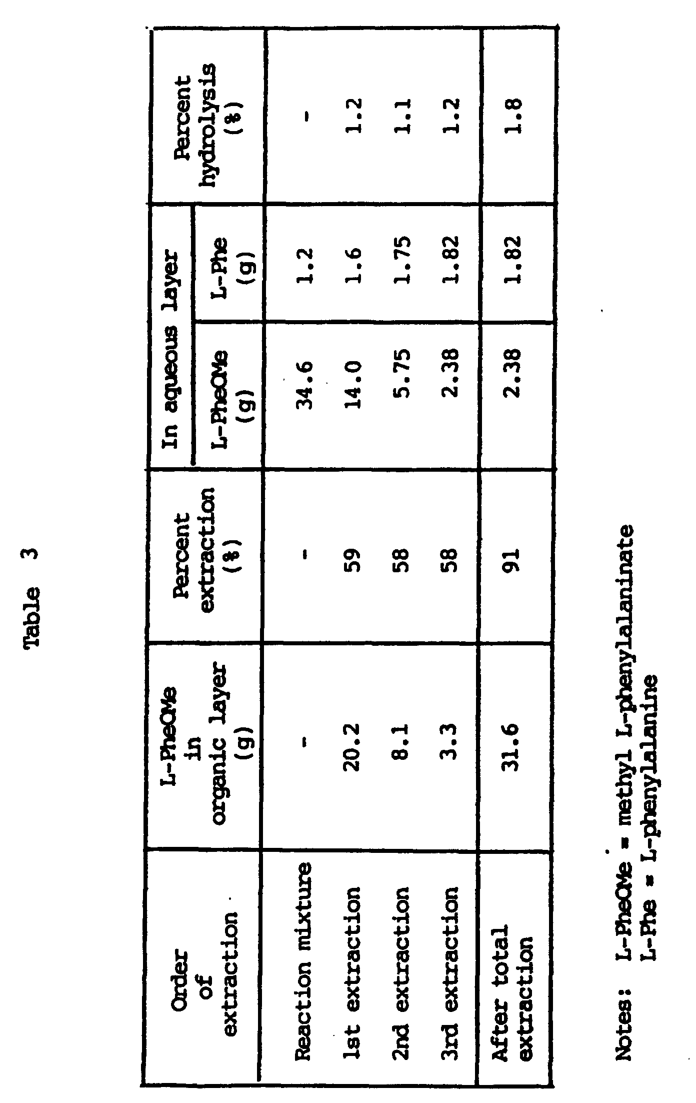

### Comparative Example 19

The same extraction procedure as in Example 12 was repeated except that the second and third extractions were carried out using no aqueous sodium hydroxide solution. The aqueous layer had pHs of 6.8 and 5.8 after the second and third extractions, respectively. Thus, the pH of the aqueous layer became lower after the second and third extractions. The results are shown in Table 4.

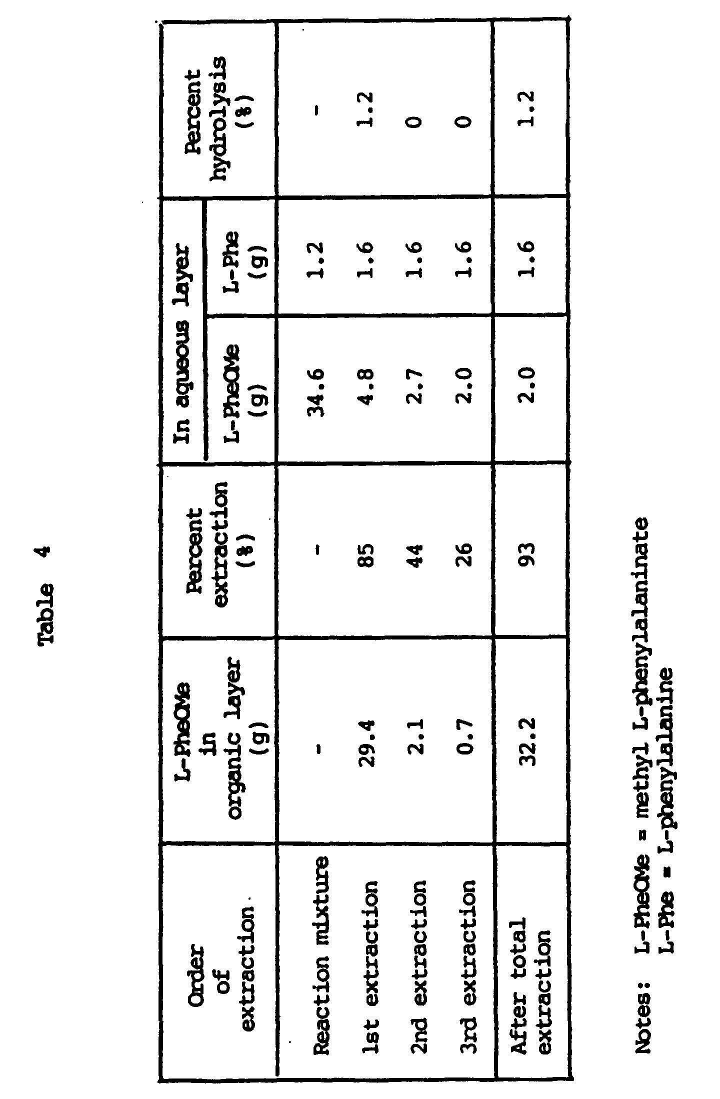

In Example 12 wherein the pH of the aqueous layer was kept at 7.5 in each extraction, the percent extraction was substantially constant (83-85%) in each extraction and, after the final extraction, 98% in total of methyl L-phenylalaninate could be extracted. Meanwhile, when the pH of the aqueous layer was not kept at 7.5 in the second and third extractions, the percent extraction decreased stepwise from 85% (first extraction) to 44% (second extraction) and 26% (third extraction), and only 93% in total of methyl L-phenylalaninate was extracted.

### Examples 14-15 and Comparative Examples 20-21

The first extraction procedure of Example 13 was repeated except that the pH of the aqueous layer was kept at 6,5, 7.0, 8.0 or 8.5. The results are shown in Table 5.

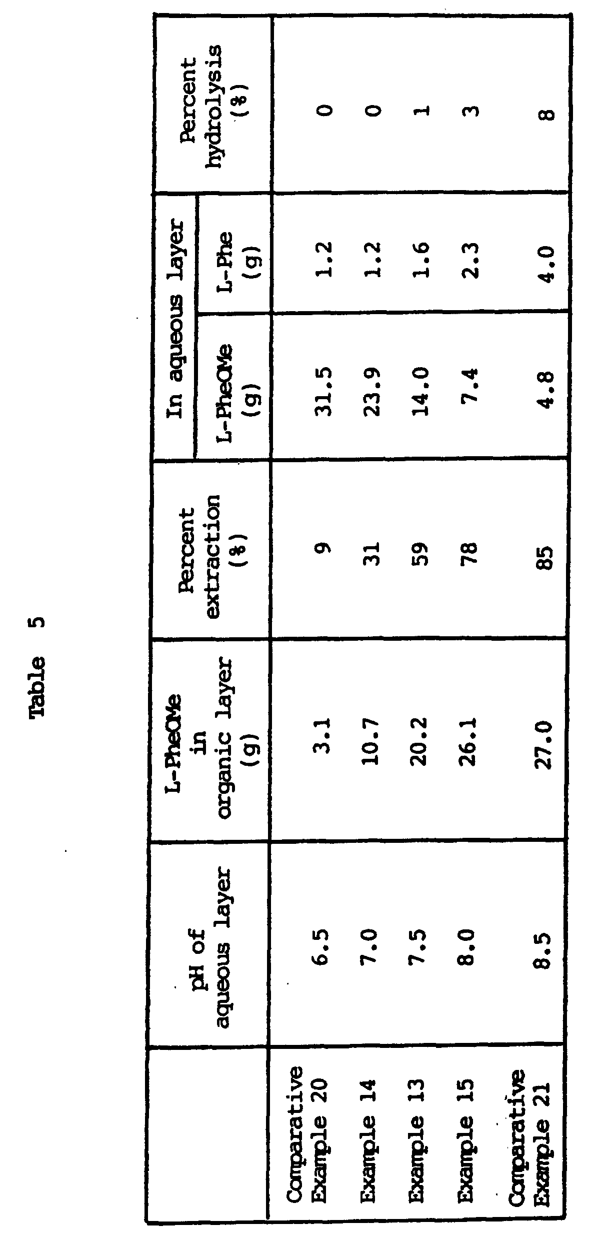

### Examples 16-17 and Comparative Example 22

The first extraction procedure of Example 12 was repeated except that the extraction was carried out at various temperature ranges each of 0-80°C, different from the range employed in Example 12. The results are shown in Table 6, together with those of Example 12.

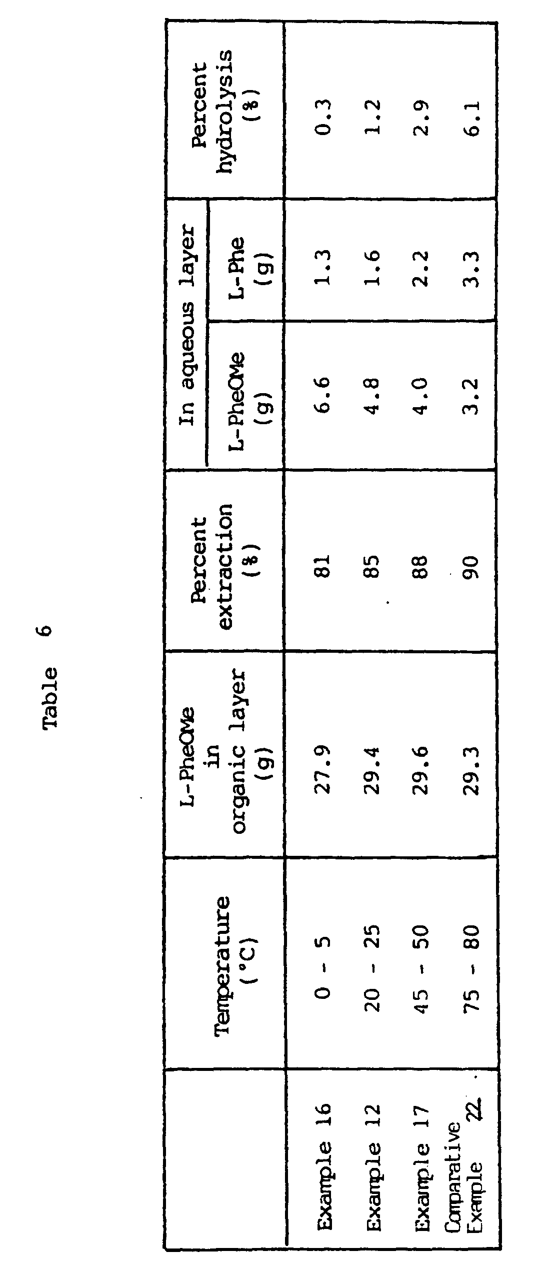

### Examples 19-21

The first extraction procedure of Example 12 was repeated except that the amount of 1,2-dichloroethane used was varied. The results are shown in Table 7, together with those of Example 12.

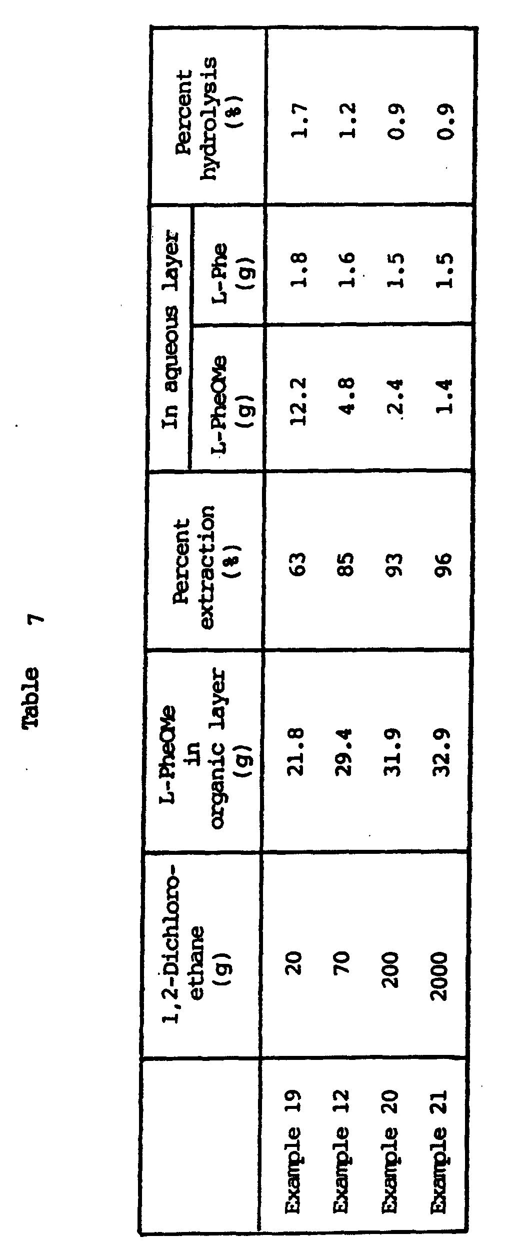

### Examples 22-25

The first extraction procedure of Example 12 was repeated except that the type of the water-insoluble solvent used was varied. The results are shown in Table 8.

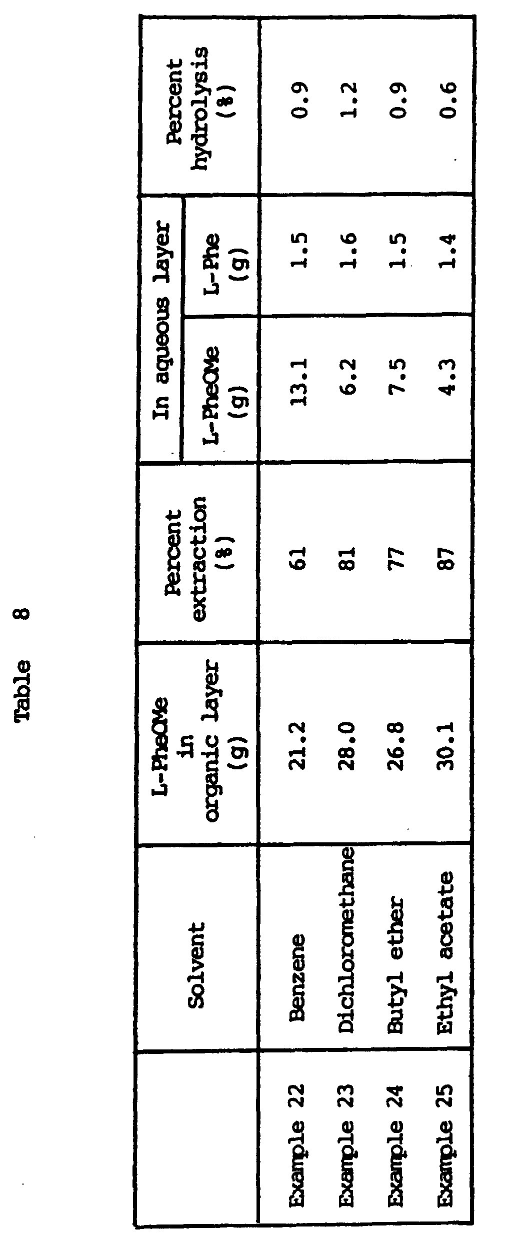

## Claims

1. A method for extracting an amino acid ester from a hydrous solution by adding a water-insoluble organic solvent to the hydrous solution containing a mineral acid salt of the amino acid ester to form an aqueous phase and a solvent phase, then adding an amount of base thereto sufficient to bring the pH to 7-8 at 0-50 °C and thereby convert only a portion of the mineral acid salt of the amino acid ester to the free base form, and promptly extracting the free base form of the amino acid ester from the aqueous phase and transferring it into the organic phase while keeping the pH of the aqueous phase 7-8 at a temperature of 0-50 °C, wherein the extraction is carried out a plurality of times, each time keeping the pH of the aqueous layer at 7-8 pH and keeping the temperature at 0-50 °C.

2. A method according to claim 1, wherein the hydrous solution is a water solution.

3. A method according to claim 1, wherein the amino acid ester is a methyl ester of an amino acid and the hydrous solution is an aqueous methanol solution.

4. A method according to claim 1, wherein the base is an inorganic base.

5. A method according to claim 1, wherein the amino acid ester is methyl L-phenylalaninate.

## Patentansprüche

1. Verfahren zur Extraktion eines Aminosäureesters aus wässriger Lösung durch Zugabe eines wasserunlöslichen organischen Lösungsmittels zu der ein Mineralsäuresalz des Aminosäureesters enthaltenden wässrigen Lösung zur Bildung einer wässrigen Phase und einer Lösungsmittelphase, sodann Zugabe einer Base in einer Menge, die ausreicht, den pH-Wert bei 0 - 50°C auf 7 - 8 einzustellen, wobei nur ein Teil des Mineralsäuresalzes des Aminosäureesters in die freie Base überführt wird, und sofortige Extraktion der freien Base des Aminosäureesters aus der wässrigen Phase und Überführung derselben in die organische Phase, wobei der pH-Wert der wässrigen Phase bei einer Temperatur von 0 - 50°C bei 7 - 8 gehalten wird, in welchem Verfahren die Extraktion mehrmals durchgeführt wird, wobei der pH-Wert der wässrigen Schicht jeweils bei 7 - 8 und die Temperatur bei 0 - 50°C gehalten wird.

2. Verfahren nach Anspruch 1, in welchem die wässrige Lösung eine Lösung in Wasser ist.

3. Verfahren nach Anspruch 1, in welchem der Aminosäureester ein Methylester einer Aminosäure und die wässrige Lösung eine wässrige methanolische Lösung ist.

4. Verfahren nach Anspruch 1, in welchem die Base eine anorganische Base ist.

5. Verfahren nach Anspruch 1, in welchem der Aminosäureester L-Phenylalaninmethylester ist.

## Revendications

1. Procédé pour extraire un ester d'acide aminé d'une solution hydratée qui consiste à ajouter un solvant organique insoluble dans l'eau à la solution hydratée contenant un sel d'acide minéral de l'ester d'acide aminé afin de former une phase aqueuse et une phase solvant, puis à y ajouter une quantité suffisante d'une base pour amener le pH à 7-8 à 0-50°C et ensuite à ne convertir qu'une partie du sel d'acide minéral de l'ester d'acide aminé en une forme de base libre et extraire promptement la forme de base libre de l'ester d'acide aminé de la phase aqueuse et la transférer dans la phase organique tout en maintenant le pH de la phase aqueuse à 7-8 à une température de 0-50°C, dans lequel l'extraction est effectuée une pluralité de fois, en maintenant chaque fois le pH de la phase aqueuse à 7-8 et en maintenant la température à 0-50°C.

2. Procédé selon la revendication 1, dans lequel la solution hydratée est une solution d'eau.

3. Procédé selon la revendication 1, dans lequel l'ester d'acide aminé est un ester méthylique d'un acide aminé et la solution hydratée est une solution aqueuse de méthanol.

4. Procédé selon la revendication 1, dans lequel la base est une base minérale.

5. Procédé selon la revendication 1, dans lequel l'ester d'acide aminé est le L-phénylalaninate de méthyle.
